# EUROPEAN PATENT SPECIFICATION

(11) **EP 2 575 637 B1**
(45) Date of publication and mention of the grant of the patent: **10.08.2016**
(21) Application number: 11724918.5
(22) Date of filing: 02.06.2011
(51) Int. Cl.: A61B 17/12, A61B 17/00

(54) **OCCLUSION DEVICE**
OKKLUSIONSVORRICHTUNG
DISPOSITIF D'OCCLUSION

(30) Priority: 02.06.2010 US 792414
(43) Date of publication of application: 10.04.2013
(73) Proprietor: Cook Medical Technologies LLC, Bloomington, IN 47404 (US)
(72) Inventor: HENDRIKSEN, Per, DK-4850 Stubbekoebing (DK); CLAUSEN, Jacob, Lund, DK-2800 Lyngby (DK)
(74) Representative: Ellis, Katherine Elizabeth Sleigh
(86) International application number: PCT/US2011/038860
(87) International publication number: WO 2011/153304

(56) References cited:
- WO-A1-96/01591
- WO-A1-2004/082532
- US-A1- 2005 137 714
- US-A1- 2007 135 826
- US-A1- 2008 249 562

## Description

### BACKGROUND OF THE INVENTION

### FIELD OF INVENTION

The present invention relates to medical devices. More particularly, the invention relates to an occlusion device for occluding a lumen of a blood vessel.

### BACKGROUND

Vascular occlusion devices are surgical implants that are placed within the vascular system of a patient. There are a number of reasons why it may be desirable to occlude a vessel. For example, the site of a stroke or other vascular accident can be treated by placing an occlusion device proximal of the site to block the flow of blood to the site, thereby alleviating leakage at the site. An aneurysm can be treated by the introduction of an occlusion device through the neck of the aneurysm. Tumours can be treated by occluding the flow of blood to a targeted site of interest.

Several known occlusion devices include a coil having fibers, threads or strands attached to the coil. Such occlusion devices act to block the flow of blood through a vessel by the formation of an embolus in the vessel. While these occlusion devices can provide effective occlusion, they suffer from the disadvantage that blood flow continues until the embolus has been formed, thus requiring additional time before effective occlusion is obtained.

Plug-style occlusion devices have also been developed. While these are intended to provide a physical barrier to blood flow, and thereby stop blood flow more quickly, known devices are generally bulky and often require thrombosis in order for reliable occlusion to be obtained.

WO 2004/082532 relates to thin film composite lamination, according to the preamble of claim 1.

US 2008/249562 discloses a septal closure device with a centring mechanism.

### BRIEF SUMMARY OF THE INVENTION

The present invention provides an improved occlusion for use in various medical procedures and a method of fabricating the occlusion device. According to an aspect of the present invention there is provided an occlusion device as specified in claim 1.

One embodiment of an occlusion device, constructed in accordance with the teachings of the present invention, generally comprises a tube having a proximal end extending to a distal end and a tubular wall defining a lumen formed therethrough. The tube defines a first collar at the proximal end and a second collar located between the proximal and distal ends. A plurality of cuts formed through the tubular wall defines a plurality of radially expandable struts. The struts are biased to a radially expanded state. The plurality of struts defines a bulbous portion extending between the first and second collars and a first conical portion extending from the second collar to the distal end of the tube. The bulbous portion is configured to expand to a first maximum diameter and the first conical portion is configured to expand to a second maximum diameter approximately equal to the first maximum diameter.

In an embodiment a radiopaque material is disposed within the second collar.

The first collar may include an attachment mechanism for attaching the device to a delivery member during delivery of the device to a patient's vessel.

According to another aspect of the present invention, there is provided a method of fabricating an occlusion device as specified in claim 15.

Further objects, features, and advantages of the present invention will become apparent from consideration of the following description and the appended claims when taken in connection with the accompanying drawings.

### BRIEF DESCRIPTION OF THE DRAWINGS

FIG. 1 is perspective view of an occlusion device in accordance with the teachings of the present invention;
FIG. 2 is a side view of the occlusion device of FIG. 1 in a partially covered condition;
FIG. 3a is a side view of the occlusion device of FIG. 2 in a collapsed state and disposed within a delivery tube during delivery of the occlusion device within a blood vessel;
FIG. 3b is a side view of the occlusion device of FIG. 2 in a partially expanded state and partially disposed within a delivery tube during delivery of the occlusion device within a blood vessel;
FIGS. 3c and 3d are side views of the occlusion device of FIG. 2 in an expanded state prior to release of the occlusion device from a deployment device during delivery of the occlusion device within a blood vessel; and

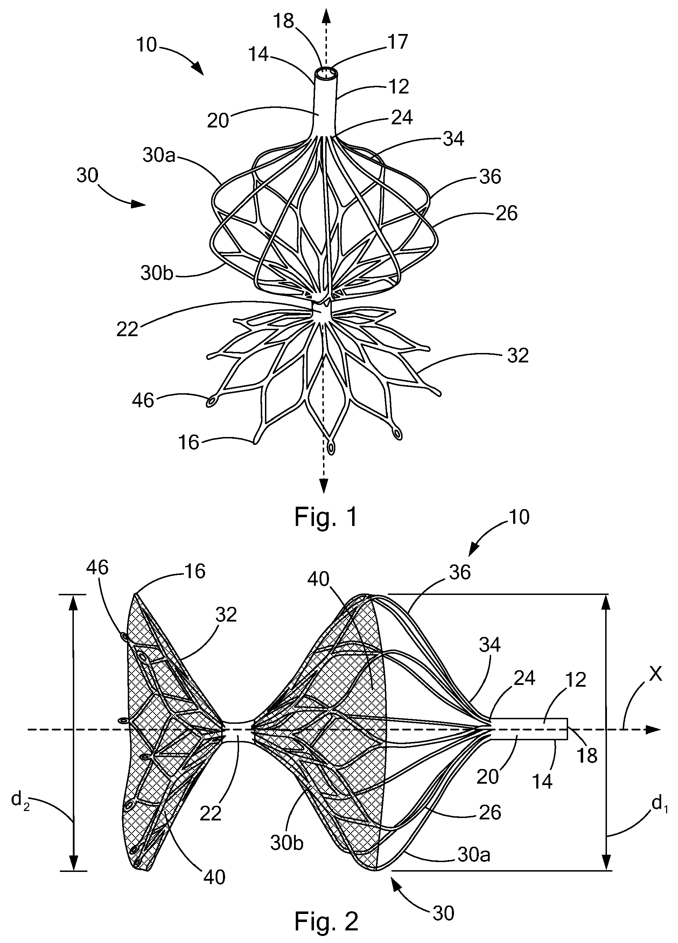

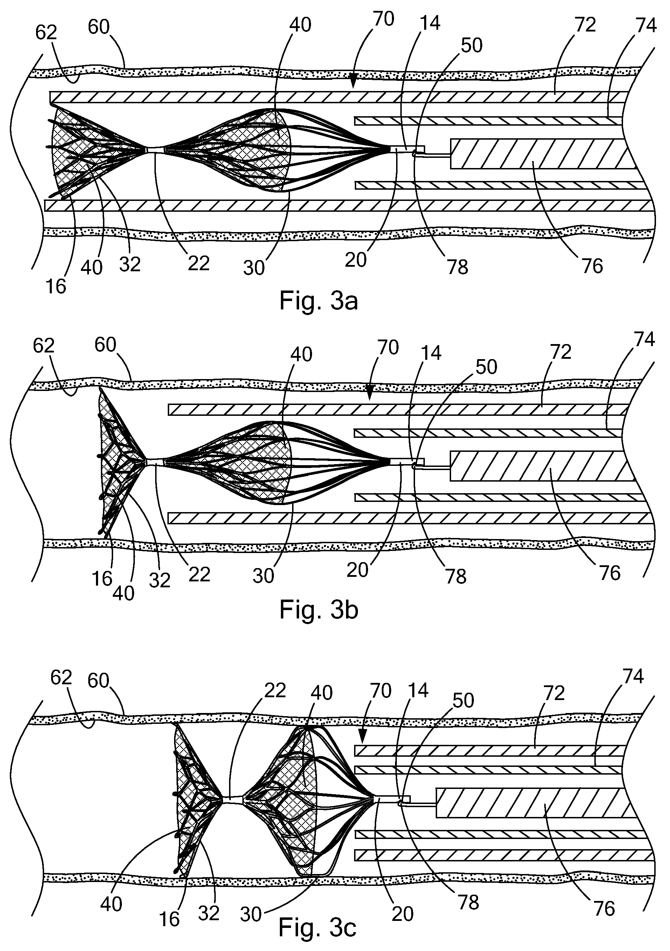

FIG. 3b is a side view of the occlusion device of FIG. 2 in a partially expanded state and partially disposed within a delivery tube during delivery of the occlusion device within a blood vessel;

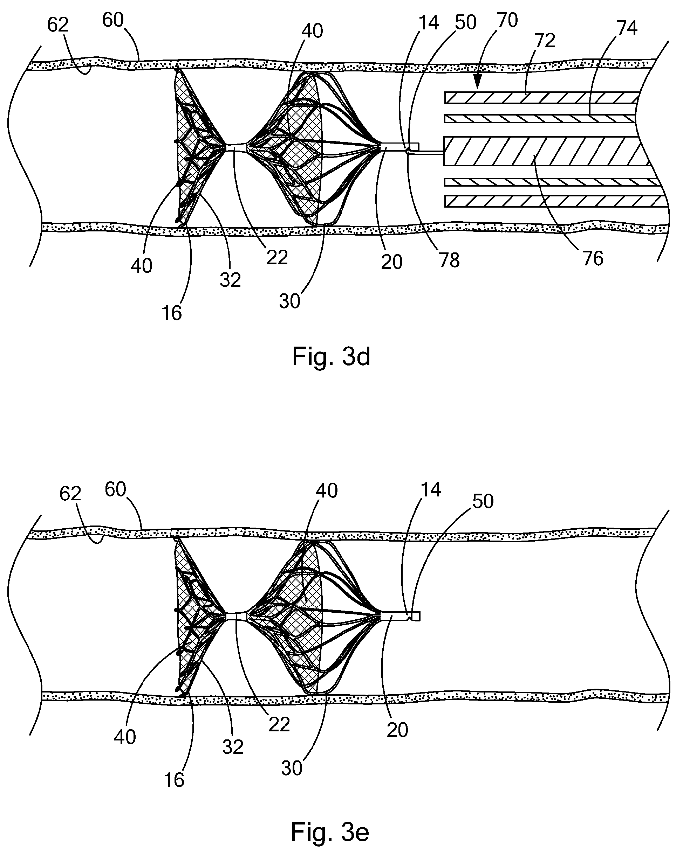

FIGS. 3c and 3d are side views of the occlusion device of FIG. 2 in an expanded state prior to release of the occlusion device from a deployment device during delivery of the occlusion device within a blood vessel; and
FIG. 3e is a side view of the occlusion device of FIG. 2 in an expanded state and fully deployed within the blood vessel;

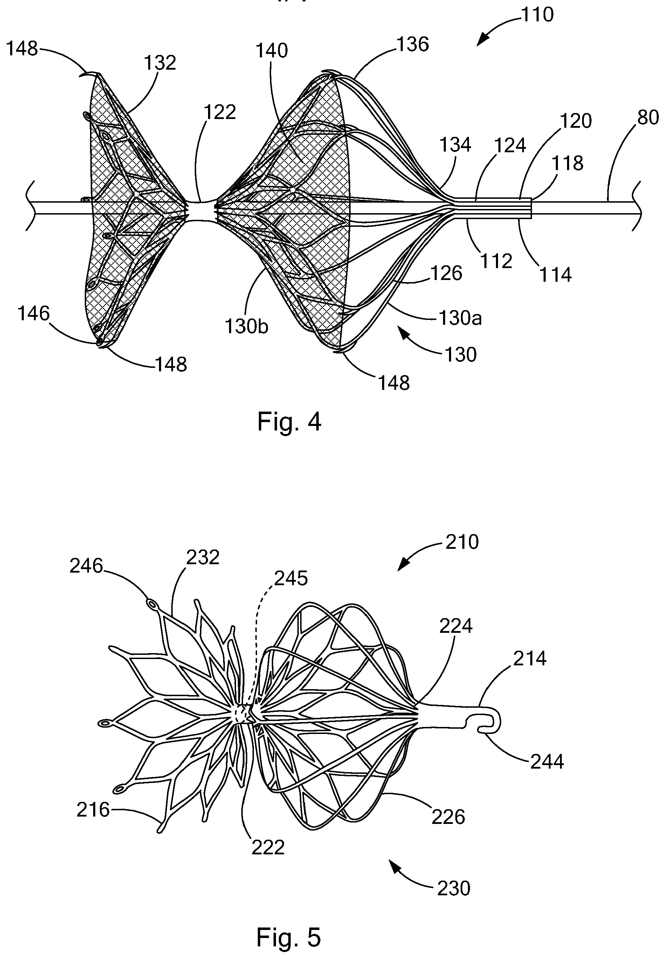

FIG. 4 is a side view of an occlusion device in accordance with another embodiment of the present invention; and
FIG. 5 is a side perspective view of an occlusion device in accordance with yet another embodiment of the present invention.

### DETAILED DESCRIPTION OF THE INVENTION

The following provides a detailed description of currently preferred embodiments of the present invention. The description is not intended to limit the invention in any manner, but rather serves to enable those skilled in the art to make and use the invention.

In this description, when referring to an introducer or deployment assembly, the term distal is used to refer to an end of a component which in use is furthest from the physician during the medical procedure, including within a patient. The term proximal is used to refer to an end of a component closest to the physician and in practice in or adjacent an external manipulation part of the deployment or treatment apparatus. Similarly, when referring to an implant such as an occlusion device the term distal is used to refer to an end of the device which in use is furthest from the physician during the medical procedure and the term proximal is used to refer to an end of the device which is closest to the physician during the medical procedure.

The present disclosure generally provides an occlusion device and an occlusion device delivery system that may be used by a physician to deliver an occlusion device into the vasculature of a patient. Referring to FIGS. 1 and 2, an occlusion device 10 is formed from a tube 12 having a proximal end 14 extending to a distal end 16. The tube 12 includes a tubular wall 17 defining a lumen 18 formed through the proximal 14 and distal ends 16. A plurality of cuts 24 is formed through the tubular wall 17 to define a plurality of radially expandable struts 26. The tube 12 includes a first non-cut portion defining a first collar 20 at the proximal end 14 and a second non-cut portion defining a second collar 22 disposed between the proximal 14 and distal ends 16.

As depicted in FIGS. 1 and 2, the plurality of struts 26 is biased to a radially expanded state in which the struts 26 define a bulbous portion 30 extending between the first 20 and second collars 22 and a flared distal conical portion 32 extending from the second collar 22 to the distal end 16 of the tube 12. The bulbous portion 30 is configured to expand to a maximum outer diameter d₁ and the distal conical portion 32 is configured to expand to a maximum outer diameter d₂, which is approximately equal to the diameter d₁ of the bulbous portion 30.

In this embodiment, the bulbous portion 30 could be said to include two conical portions 30a and 30b joined together such that the device 10 includes a total of three conical portions. Each conical portion 30a, 30b and 32 could be said to include a base and an apex. For example, the conical portion 30a of the bulbous portion 30 includes an apex at about the first collar 20 and extends to a base at about where the maximum outer diameter of the expanded bulbous portion 30 is approximately d₁. The conical portion 30b of the bulbous portion 30 includes a base at about where the maximum outer diameter of the expanded bulbous portion 30 is approximately d₁ and extends to an apex at about the second collar 22. Thus, the conical portions 30a and 30b are joined together at their respective bases to define the bulbous portion 30. In addition, the distal conical portion 32 includes an apex at about the second collar 32 and extends to a base at the most distal end 16 of the tube 12. As shown in FIGS. 1 and 2, the bulbous portion 30 includes curved or rounded portions at the widest diameter in the expanded, non-deployed condition. The term "bulbous portion," however, may further include embodiments in which the device 10 has a flattened portion or strip between the two conical portions 30a and 30b in the expanded, non-deployed condition.

As shown, the conical portions 30a and 30b are each defined by a different pattern such that about one half of the bulbous portion 30 has one pattern and about another half of the bulbous portion 30 has a different pattern. Preferably, the distal conical portion 32 is defined by substantially the same pattern as its adjacent conical portion 30b of the bulbous portion 30 such that they are essentially mirror images of one another.

In this embodiment, the conical portion 30a is defined by a first set of cuts 24 formed through the tubular wall 17. The first set of cuts 24 extend from the first collar 20 (i.e., the apex of the conical portion 30a) to about where the expanded bulbous portion 30 defines the maximum outer diameter d₁ (i.e., the base of the conical portion 30a) to define a first pattern. The conical portion 30b is defined by a second set of cuts 24 formed through the tubular wall 17. The second set of cuts 24 extend from about where the expanded bulbous portion 30 defines the maximum outer diameter d₁ (i.e., the base of the conical portion 30b) to the second collar 22 (i.e., the apex of the conical portion 30b) to define a second pattern. The distal conical portion 32 is defined by a third set of cuts 24 formed through the tubular wall 17. The third set of cuts 24 extend from the second collar 32 (i.e., the apex of the distal conical portion 32) to the distal most end 16 of the tube 12 to define a third pattern.

As provided above, the conical portion 30b and the distal conical portion 32 are essentially mirror images of each other. Thus, the second pattern of the conical portion 30b and the third pattern of the distal conical portion 32 are substantially the same, while the first pattern of the conical portion 30a is unique in comparison.

As best illustrated in FIGS. 1 and 2, the first pattern of the conical portion 30a defines a plurality of struts 26 extending arcuately from the first collar 20 to the conical portion 30b. In this embodiment, the struts 26 of the conical portion 30a include a first curved portion 34 and a second curved portion 36. The first curved portion extends from the first collar 20 in a direction away from the longitudinal axis X of the tube 12 and the second curved portion 36 extends from the first curved portion 34 in a direction toward the longitudinal axis X. As shown, the second and third patterns of respective conical portions 30b and 32 define a plurality of diamond shapes. In this embodiment, as the struts 26 of the conical portion 30a extend from the apex of the conical portion 30a (i.e., the first collar 20) toward the base of the conical portion 30a, they do not contact any other struts 26 of the conical portion 30a. Whereas, each of the struts 26 of the conical portions 30b and 32 contacts another strut 26 to define the plurality of diamond shapes.

The cuts 24 formed through the tubular wall 17 of the tube 12 are preferably formed by laser-cutting the tube 12. The laser-cut tube 12 is of a material that allows the device 10 to be self-expanding. For example, the tube 12 may be formed from a shape-memory alloy (such as Nitinol), a shape-memory polymer, or may be formed from other self-expandable materials, such as spring steel.

As illustrated in FIG. 2, the device 10 includes an occluding membrane 40, such as graft material. The graft material may be PTFE or electrospun PTFE, for example. The occluding membrane 40 may be formed from other suitable materials known or contemplated by one of ordinary skill in the art, including but not limited to Thorolon®, Dacron®, Gore-tex®, PET and the like. As shown, the occluding membrane 40 is attached to the distal most conical portion 30b of the bulbous portion 30 and the distal conical portion 32. In this embodiment, the occluding membrane 40 is attached to an interior surface of the struts 26 of each of the conical portion 30b and the distal conical portion 32. The occluding membrane 40 may be attached to the struts 26 by any suitable means known in the art, including but not limited to bonding with silicon adhesive.

Instead of a membrane 40, fibers could be provided on or within the occlusion device 10. These fibers may be silk, nylon, PET, or electrospun PTFE, for example.

In this embodiment, the substantially similar second and third patterns are preselected so that the device 10 provides effective occlusion regardless of the direction of blood flow, from right to left or from left to right in FIG. 3e. Preferably, blood flows from right to left in FIG. 3e. The first pattern is preselected so that the space between adjacent struts 26 of the conical portion 30a is larger to allow blood to flow to the center of the device 10. Allowing the blood to flow into the center of the device 10 creates turbulence around the device 10, thereby enhancing the rate of blood occlusion.

The device 10 is sized so that in its expanded configuration its outer diameter at its widest point (e.g., d₁ and d₂) is greater than that of the blood vessel 60 into which it is to be placed. For example, in a vessel 60 having a diameter of about 8 mm, the maximum outer diameter d₁ of the bulbous portion 30 and the maximum outer diameter d₂ of the distal conical portion 32 are preferably about 9 mm or about 10 mm. This results in some compression of the device *in situ.*

Thus, the device 10 preferably has substantially the same oversizing in both the bulbous portion 30 and the distal conical portion 32. The term "oversizing" can be described with the following example: a device having an outer diameter of about 10 mm implanted into a blood vessel with a diameter of about 8 mm results in an oversizing of about 2 mm. In this embodiment, the same oversizing of the bulbous portion 30 and the distal conical portion 32 results in about the same force exerted by the bulbous portion 30 and the distal conical portion 32 against the vessel wall 62 during expansion of the device 10 within the blood vessel 60. Thus, the vessel wall 62 exerts a more even distribution of return force upon the device 10 during expansion thereof compared to a device in which the maximum diameters d₁ and d₂ are not equal to one another.

The degree of oversize will generally be less than about 50%, or more preferably about 25% or less. The degree of oversize desired depends on many factors, such as in which blood vessel the device is to be located (for example, artery or vein), the rate of blood flow at the desired location, and even on the medical condition of the patient. For some implementations, there may be no oversize at all.

As shown, the distal conical portion 32 includes a plurality of radiopaque markers 46. The radiopaque markers 46 may be made from gold, tantalum, palladium, platinum or any other suitable material recognized by one of ordinary skill in the art.

FIGS. 3a-e depict deployment of the occlusion device 10 within a blood vessel 60 via a delivery system 70. The delivery system 70 includes an outer sheath 72, an inner sheath 74 housed within the lumen of the outer sheath 72, and a pusher member 76 housed within the lumen of the inner sheath 74. In this embodiment, the first collar 20 of the occlusion device 10 includes a notch 50 or some type of aperture for engaging with the pusher member 76. The pusher member 76 includes an attachment member 78 at a distal end thereof for retaining the occlusion device 10. For example, as shown, the pusher member 76 includes a hook 78 for engaging in the notch 50. Alternatively, the attachment member 78 may include any other type of attachment mechanism, such as one that can be used over a guide wire as described in U.S. Provisional Application No. 61/072903.

Referring to FIG. 3a, the delivery system 70 is introduced within the blood vessel 60 with the occlusion device 10 in the undeployed, collapsed state. As shown, the pusher member 76 and the proximal end 14 of the occlusion device 10 are housed within the inner sheath 74, all of which are housed within the outer sheath 72. The outer sheath 72 extends to the distal end 16 of the occlusion device 10 to maintain the occlusion device 10 in the compressed, collapsed state for delivery.

Once the delivery system 70 is properly positioned at a desired location within the blood vessel 60, the outer sheath 72 is withdrawn in a proximal direction to allow the distal conical portion 32 of the occlusion device 10 to expand within the blood vessel 60 as can be seen in FIG. 3b. In the event that blood flows in the direction from left to right in FIG. 3b, expansion of only the distal conical portion 32 of the occlusion device 10 results in immediate occlusion of the blood vessel 60 even though the occlusion device 10 has not been fully deployed. This immediate occlusion is assisted by blood flow in the direction of left to right in FIG. 3b, which pushes against the base of the distal conical portion 32.

At this stage of deployment, the physician can readily monitor the positioning of the occlusion device 10 using the radiopaque markers 46, and if necessary, withdraw the occlusion device 10 back into the outer sheath 72 to allow repositioning.

Once the distal conical portion 32 of the occlusion device 10 has been properly positioned, the outer sheath 72 can be withdrawn further to allow the bulbous portion 30 to expand. This is illustrated in FIG. 3c. Engagement of the distal conical portion 32 within the vessel 60 prior to release of the remainder of the occlusion device 10 helps to avoid migration of the occlusion device 10 during deployment. It can be seen that the bulbous portion 30 is slightly compressed by the blood vessel wall 62. This is because it is dimensioned such that when fully expanded it is wider than the diameter of the blood vessel 60 into which it is to be deployed. This assists in obtaining a good seal between the occlusion device 10 and the blood vessel wall 62.

In the next stage of deployment shown in FIG. 3d, the inner sheath 74 and the outer sheath 72 are withdrawn so that the entirety of the occlusion device 10 is exposed. At this stage, the occlusion device 10 is retained by the attachment member 78 of the pusher member 76. However, the physician may still recover the occlusion device 10 within the outer sheath 72 to reposition if necessary.

In the final stage of deployment, the attachment member 78 of the pusher member 76 is disengaged from the notch 50 at the proximal end 14 of the occlusion device 10. The pusher member 76 and the inner sheath 74 within the outer sheath 72 are fully withdrawn from the blood vessel 60, leaving only the occlusion device 10 fully deployed within the blood vessel 60, as shown in FIG. 3e.

During deployment, contrast injection can be used to verify the position of the occlusion device 10. If adjustment is required, the device can be retracted back into the inner and outer sheaths 74 and 72 to be relocated.

While the above method describes a preferred method of deployment, a person of ordinary skill in the art will appreciate that other deployment methods are possible. For example, deployment of the occlusion device 10 may be over a guide wire 80 (as shown and further described with respect to the embodiment in FIG. 4).

The embodiments of the present invention have many advantages. The occlusion device 10 has a low profile because it is cut out of a very small tube. Laser-cutting allows compression of the occlusion device 10 back to a very thin tube, which is ideal for delivery. The bulbous portion 30 and the distal conical portion 32 of the occlusion device 10 assist in maintaining the occlusion device 10 in the correct orientation such that the longitudinal axis X of the device 10 is aligned with the axis of the blood vessel 60 at the point of occlusion. Furthermore, the planes of the widest parts of the bulbous portion 30 and distal conical portion 32 are substantially perpendicular to the axis of the blood vessel 60. The diamond shapes formed by the struts 26 in the distal conical portion 32 and the conical portion 30b of the bulbous portion 30 assist in retractability of the occlusion device 10 because they provide a continuous structure that would not snag on the distal end of the delivery system 70. Moreover, the embodiments of the present invention provide faster occlusion than prior art coil embolization devices. Additionally, the occlusion device 10 has a lower profile and is shorter and less bulky than prior art vascular plugs.

FIG. 4 illustrates an embodiment of the present invention having a description similar to that of FIG. 1 and in which similar components are denoted by similar reference numeral increased by 100. As shown, the bulbous portion 130 of the occlusion device 110 includes a plurality of barbs 148 that extend radially outwardly from the occlusion device 110 at around the widest diameter of the bulbous portion 130. The barbs 148 aid in attachment of the occlusion device 110 to the vessel wall 62 of the blood vessel 60. Similarly, the distal conical portion 132 may include barbs 148 to further aid in attachment within the blood vessel 60. The barbs 148 may be particularly useful if the occlusion device 110 is not dimensioned larger than the diameter of the blood vessel. The barbs 148 can be of a type designed to cause irritation to the blood vessel wall 62, which can provoke tissue ingrowth (stenosis). This can assist in providing improved occlusion.

Further illustrated in FIG. 4, the cuts 124 may extend all the way through the proximal end 114 of the tube 112 such that the first collar 20 is no longer defined by a non-cut portion. In this embodiment, the struts 126 defined by the cuts 124 of the first collar 120 may be spread apart for easy placement of the occluding membrane 140 within the interior of the bulbous portion 130. The proximal ends of the struts 126 are then adhered, glued or soldered back together to form the first collar 120 defining the lumen 118.

In this embodiment, a guide wire 80 is shown extending through the lumen 118 of the tube 112. Deployment of the occlusion device 110 over the guide wire 80 facilitates precise delivery of the occlusion device 110 within the blood vessel 60.

FIG. 5 illustrates an embodiment of the present invention having a description similar to that of FIG. 1 and in which similar components are denoted by similar reference numeral increased by 200. As shown, the proximal end 214 of the occluding device 210 includes a hook 244 for assisting with delivery. The hook 244 may be formed by cutting a hook 244 shape into the tube 212. Alternatively, the hook may be welded onto the proximal end 214 of the tube 212.

Further illustrated in FIG. 5, is a radiopaque marker 245 disposed within the second collar 222 to aid in visualization of the occlusion device 210 during delivery. The occlusion device 210 preferably includes an occluding membrane as described with respect to FIG. 2, but is not shown in FIG. 5 for illustration purposes.

As a person skilled in the art will readily appreciate, the above description is meant as an illustration of the implementation of the principles of this invention. This description is not intended to limit the scope or application of this invention in that the invention is susceptible to modification variation and change, without departing from the scope of this invention, as defined in the following claims.

## Claims

1. An occlusion device (10;110;210) comprising:
a tube (12;112;212) having a proximal end (14;114;214) extending to a distal end (16) and a tubular wall (17) defining a lumen (18;118) formed therethrough, the tube defining a first collar (20;120) at the proximal end and a second collar (22;222) disposed between the proximal and distal ends, a plurality of cuts (24;124) formed through the tubular wall defining a plurality of radially expandable struts (26;126; 226), the plurality of struts being biased to a radially expanded state, wherein the plurality of struts defines a bulbous portion (30;130) extending between the first and second collars and a first conical portion (32;132) extending from the second collar to the distal end of the tube;
wherein the first conical portion is defined by a first set of cuts (24;124) formed through the tubular wall in a first pattern;
wherein the bulbous portion includes a second conical portion (30b) and a third conical portion (30a), wherein the second conical portion is defined by a second set of cuts formed through the tubular wall in a second pattern, and the third conical portion is defined by a third set of cuts formed through the tubular wall in a third pattern;
wherein the second conical portion is adjacent to both the first conical portion and the third conical portion;
wherein the second and third patterns are different such that the struts of the second and third conical portions of the bulbous portion form different patterns, said occlusion device being **characterized in that** the third pattern is preselected so that the space between adjacent struts (26; 126; 226) of the third conical portion (30a) is larger than that of the second conical portion.

2. A device (10;110;210) as claimed in claim 1, wherein the bulbous portion (30;130) is configured to expand to a first maximum diameter (d₁) and the first conical portion (32;132) is configured to expand to a second maximum diameter (d₂) equal to the first maximum diameter.

3. A device (10;110;210) as claimed in claim 1, wherein the first conical portion (32;132) extends from a first apex at the second collar (22;222) to a first base at the distal end (16) of the tube (12;112;212).

4. A device as claimed in claim 3, wherein the second conical portion (30b) has a second apex at the second collar and the third conical portion (30a) has a third apex at the first collar, each of the second and third conical portions extending from their respective second and third apices to respective second and third bases joined together.

5. A device (10;110;210) as claimed in claim 1, wherein the second pattern of the second conical portion (30b) is a mirror image of the first pattern of the first conical portion (32;132), and wherein the third pattern is different from both the first and second patterns.

6. A device (10;110;210) as claimed in claim 4, wherein the third pattern of the third conical portion (30a) defines struts (26;126) extending arcuately between the first collar (20;120) and the second conical portion (30b), each of the struts of the third conical portion avoiding contact with other struts of the third conical portion as the struts extend from the third apex towards the third base.

7. A device (10;110;210) as claimed in claim 1, wherein the first collar (20;120) is defined by a first non-cut portion of the tube (12;112;212) and the second collar (22;222) is defined by a second non-cut portion of the tube, the first set of cuts (24;124) extending from the second collar to the distal end (16) of the tube, the second set of cuts extending from the second collar to the third conical portion (30a), and the third set of cuts extending from the second conical portion (30b) to the first collar.

8. A device as claimed in claim 1, wherein the second collar (22;222) is defined by a non-cut portion of the tube (12;112;212), the first set of cuts (24) extending from the second collar to the distal end (16) of the tube, the second set of cuts extending from the second collar to the third conical portion (30a), and the third set of cuts extending from the second conical portion (30b) to the proximal end (14;114;214) of the tube, wherein the struts (26) formed by the third set of cuts are brought together at proximal ends thereof to define the first collar (20;120).

9. A device as claimed in claim 1, further comprising an occluding membrane (40;140) attached to the struts (26) at the first and second conical portions (32,30b).

10. A device as claimed in claim 9, wherein the occluding membrane (40;140) is attached to an interior surface of the struts at the first and second conical portions (32,30b).

11. A device (10;110;210) as claimed in claim 4, wherein, in the radially expanded state, the first base of the first conical portion (32;132) defines a first diameter (d₁), the second base of the second conical portion (30b) defines a second diameter (d₂), and the third base of the third conical portion (30a) defines a third diameter (d₂), wherein the first, second, and third diameters are equal to one another such that the bulbous portion (30) and the first conical portion have the same diameter.

12. A device (10;110;210) as claimed in claim 1, wherein the first and second patterns of respective first and second conical portions (32;132,30b) are defined by a plurality of diamond shapes formed by the plurality of struts (26) of each of the first and second conical portions (32;132,30b).

13. A device (10;110;210) as claimed in claim 1, wherein the device is deployable over a guide wire (80), the lumen (18;118) of the tube (12;112;212) configured to receive the guide wire.

14. A device (210) as claimed in claim 1, wherein the first collar includes an attachment mechanism (244) for attaching the device to a delivery member during delivery of the device to a patient's vessel.

15. A method of fabricating an occlusion device (10;110;210) comprising:
cutting a plurality of cuts (24) through a portion of a tubular wall (17) of a tube (12;112;212) having a proximal end (14;114;214) and a distal end (16), the tubular wall defining a lumen (18;118) formed through the proximal and distal ends of the tube, wherein cutting a plurality of cuts through the tubular wall defines a plurality of radially expandable struts; and
expanding the plurality of struts to form a frame having a first collar (20;120) at the proximal end of the tube, a bulbous portion (30) extending between the first collar and a second collar (22;222) located between the proximal and distal ends of the tube, and a flared portion (32;132) extending from the second collar to the distal end of the tube, wherein expanding the plurality of struts includes expanding the bulbous portion and the flared portion to the same maximum diameter (d₁,d₂);
wherein cutting a plurality of cuts (24) through the tubular wall (17) includes cutting a first set of cuts through a first portion of the tubular wall, cutting a second set of cuts through a second portion of the tubular wall, and cutting a third set of cuts through a third portion of the tubular wall, the first set of cuts being formed in a first pattern, the second set of cuts being formed in a second pattern, and the third set of cuts being formed in a third pattern, the flared portion (32;132) being defined by the third portion with the third set of cuts, the bulbous portion (30) being defined by the first and second portions, the first portion with the first set of cuts forming a first conical portion (30a) and the second portion with the second set of cuts forming a second conical portion (30b), each of the first and second conical portions having respective first and second apices and first and second bases, the first and second conical portions being joined at the first and second bases, the first collar defining the first apex of the first conical portion and the second collar defining the second apex of the second conical portion, wherein the second and third patterns are mirror images of each other, and wherein the first pattern is different from the second and third patterns and is preselected so that the space between adjacent struts of the first conical portion is larger than that of the second conical portion.

## Patentansprüche

1. Okklusionsvorrichtung (10; 110; 210), welche Folgendes umfasst:
einen sich von einem proximalen Ende (14; 114; 214) zu einem distalen Ende (16) erstreckenden Schlauch (12; 112; 212), wobei eine schlauchförmige Wand (17) ein durchgehendes Lumen (18; 118) definiert, wobei der Schlauch einen ersten Kragen (20; 120) am proximalen Ende und einen zwischen dem proximalen und dem distalen Ende angeordneten zweiten Kragen (22; 222) definiert, wobei mehrere Schnitte (24; 124) durch die schlauchförmige Wand gebildet sind, die mehrere radial expandierbare Stege (26; 126; 226) definieren, wobei die mehreren Stege in einen radial expandierten Zustand vorgespannt sind, wobei die mehreren Stege einen bauchigen Teil (30; 130), der sich zwischen dem ersten und dem zweiten Kragen erstreckt, und einen ersten konischen Teil (32; 132) definieren, der sich vom zweiten Kragen zum distalen Ende des Schlauchs erstreckt;
wobei der erste konische Teil durch einen ersten Satz von Schnitten (24; 124) definiert ist, die durch die schlauchförmige Wand in einem ersten Muster gebildet sind;
wobei der bauchige Teil einen zweiten konischen Teil (30b) und einen dritten konischen Teil (30a) aufweist, wobei der zweite konische Teil durch einen zweiten Satz von Schnitten definiert ist, die durch die schlauchförmige Wand in einem zweiten Muster gebildet sind, und wobei der dritte konische Teil durch einen dritten Satz von Schnitten definiert ist, die durch die schlauchförmige Wand in einem dritten Muster gebildet sind;
wobei der zweite konische Teil an sowohl den ersten konischen Teil als auch den dritten konischen Teil angrenzend ist;
wobei das zweite und das dritte Muster unterschiedlich sind, so dass die Stege des zweiten und des dritten konischen Teils des bauchigen Teils unterschiedliche Muster bilden, wobei die Okklusionsvorrichtung **dadurch gekennzeichnet ist, dass** das dritte Muster derart vorausgewählt ist, dass der Raum zwischen benachbarten Stegen (26; 126; 226) des dritten konischen Teils (30a) größer ist als der des zweiten konischen Teils.

2. Vorrichtung (10; 110; 210) nach Anspruch 1, wobei der bauchige Teil (30; 130) konfiguriert ist, auf einen ersten maximalen Durchmesser (d₁) zu expandieren, und der erste konische Teil (32; 132) konfiguriert ist, auf einen zweiten maximalen Durchmesser (d₂) zu expandieren, der gleich dem ersten maximalen Durchmesser ist.

3. Vorrichtung (10; 110; 210) nach Anspruch 1, wobei der erste konische Teil (32; 132) sich von einem ersten Scheitelpunkt am zweiten Kragen (22; 222) zu einer ersten Basis am distalen Ende (16) des Schlauchs (12; 112; 212) erstreckt.

4. Vorrichtung nach Anspruch 3, wobei der zweite konische Teil (30b) einen zweiten Scheitelpunkt am zweiten Kragen aufweist und der dritte konische Teil (30a) einen dritten Scheitelpunkt am ersten Kragen aufweist, wobei der zweite und der dritte konische Teil, die sich jeweils von ihrem zweiten bzw. dritten Scheitelpunkt zur zweiten bzw. dritten Basis erstrecken, miteinander verbunden sind.

5. Vorrichtung (10; 110; 210) nach Anspruch 1, wobei das zweite Muster des zweiten konischen Teils (30b) ein Spiegelbild des ersten Musters des ersten konischen Teils (32; 132) ist und wobei das dritte Muster von sowohl dem ersten als auch dem zweiten Muster unterschiedlich ist.

6. Vorrichtung (10; 110; 210) nach Anspruch 4, wobei das dritte Muster des dritten konischen Teils (30a) Stege (26; 126) definiert, die sich bogenförmig zwischen dem ersten Kragen (20; 120) und dem zweiten konischen Teil (30b) erstrecken, wobei jeder der Stege des dritten konischen Teils einen Kontakt mit anderen Stegen des dritten konischen Teils vermeidet, wenn die Stege sich vom dritten Scheitelpunkt zur dritten Basis erstrecken.

7. Vorrichtung (10; 110; 210) nach Anspruch 1, wobei der erste Kragen (20; 120) durch einen ersten ungeschnittenen Teil des Schlauchs (12; 112; 212) definiert ist und der zweite Kragen (22; 222) durch einen zweiten ungeschnittenen Teil des Schlauchs definiert ist, wobei der erste Satz von Schnitten (24; 124) sich vom zweiten Kragen zum distalen Ende (16) des Schlauchs erstreckt, wobei der zweite Satz von Schnitten sich vom zweiten Kragen zum dritten konischen Teil (30a) erstreckt und wobei der dritte Satz von Schnitten sich vom zweiten konischen Teil (30b) zum ersten Kragen erstreckt.

8. Vorrichtung nach Anspruch 1, wobei der zweite Kragen (22; 222) durch einen ungeschnittenen Teil des Schlauchs (12; 112; 212) definiert ist, wobei der erste Satz von Schnitten (24) sich vom zweiten Kragen zum distalen Ende (16) des Schlauchs erstreckt, wobei der zweite Satz von Schnitten sich vom zweiten Kragen zum dritten konischen Teil (30a) erstreckt und wobei der dritte Satz von Schnitten sich vom zweiten konischen Teil (30b) zum proximalen Ende (14; 114; 214) des Schlauchs erstreckt, wobei die durch den dritten Satz von Schnitten gebildeten Stege (26) an deren proximalen Enden zusammengebracht sind, um den ersten Kragen (20; 120) zu definieren.

9. Vorrichtung nach Anspruch 1, welche ferner eine Okklusionsmembran (40; 140) umfasst, die an den Stegen (26) am ersten und am zweiten konischen Teil (32, 30b) angebracht ist.

10. Vorrichtung nach Anspruch 9, wobei die Okklusionsmembran (40; 140) an einer Innenfläche der Stege am ersten und am zweiten konischen Teil (32, 30b) angebracht ist.

11. Vorrichtung (10; 110; 210) nach Anspruch 4, wobei im radial expandierten Zustand die erste Basis des ersten konischen Teils (32; 132) einen ersten Durchmesser (d₁) definiert, die zweite Basis des zweiten konischen Teils (30b) einen zweiten Durchmesser (d₂) definiert und die dritte Basis des dritten konischen Teils (30a) einen dritten Durchmesser (d₂) definiert, wobei der erste, der zweite und der dritte Durchmesser einander gleich sind, so dass der bauchige Teil (30) und der erste konische Teil den gleichen Durchmesser aufweisen.

12. Vorrichtung (10; 110; 210) nach Anspruch 1, wobei das erste und das zweite Muster des ersten bzw. zweiten konischen Teils (32; 132, 30b) durch mehrere Diamantformen definiert sind, die durch die mehreren Stege (26) des ersten als auch des zweiten konischen Teils (32; 132, 30b) gebildet sind.

13. Vorrichtung (10; 110; 210) nach Anspruch 1, wobei die Vorrichtung über einen Führungsdraht (80) ausfaltbar ist, wobei das Lumen (18; 118) des Schlauchs (12; 112; 212) konfiguriert ist, den Führungsdraht aufzunehmen.

14. Vorrichtung (210) nach Anspruch 1, wobei der erste Kragen einen Befestigungsmechanismus (244) zum Anbringen der Vorrichtung an einem Zuführungselement während der Zufuhr der Vorrichtung in ein Gefäß eines Patienten aufweist.

15. Verfahren zum Herstellen einer Okklusionsvorrichtung (10; 110; 210), welches Folgendes umfasst:
Schneiden von mehreren Schnitten (24) durch einen Teil einer schlauchförmigen Wand (17) eines Schlauchs (12; 112; 212) mit einem proximalen Ende (14; 114; 214) und einem distalen Ende (16), wobei die schlauchförmige Wand ein Lumen (18; 118) definiert, das durch das proximale und das distale Ende des Schlauchs gebildet wird, wobei das Schneiden von mehreren Schnitten durch die schlauchförmige Wand mehrere radial expandierbare Stege definiert; und
Expandieren der mehreren Stege, um einen Rahmen mit einem ersten Kragen (20; 120) am proximalen Ende des Schlauchs zu bilden, wobei ein bauchiger Teil (30) sich zwischen dem ersten Kragen und einem zweiten Kragen (22; 222) zwischen dem proximalen und dem distalen Ende des Schlauchs erstreckt, und ein aufgeweiteter Teil (32; 132) sich vom zweiten Kragen zum distalen Ende des Schlauchs erstreckt, wobei das Expandieren der mehreren Stege das Expandieren des bauchigen Teils und des aufgeweiteten Teils auf den gleichen maximalen Durchmesser (d₁, d₂) umfasst;
wobei das Schneiden von mehreren Schnitten (24) durch die schlauchförmige Wand (17) das Schneiden eines ersten Satzes von Schnitten durch einen ersten Teil der schlauchförmigen Wand, das Schneiden eines zweiten Satzes von Schnitten durch einen zweiten Teil der schlauchförmigen Wand und das Schneiden eines dritten Satzes von Schnitten durch einen dritten Teil der schlauchförmigen Wand umfasst, wobei der erste Satz von Schnitten in einem ersten Muster gebildet wird, der zweite Satz von Schnitten in einem zweiten Muster gebildet wird und der dritte Satz von Schnitten in einem dritten Muster gebildet wird, wobei der aufgeweitete Teil (32; 132) durch den dritten Teil mit dem dritten Satz von Schnitten definiert wird, wobei der bauchige Teil (30) durch den ersten und den zweiten Teil definiert wird, wobei der erste Teil mit dem ersten Satz von Schnitten einen ersten konischen Teil (30a) bildet und der zweite Teil mit dem zweiten Satz von Schnitten einen zweiten konischen Teil (30b) bildet, wobei der erste als auch der zweite konische Teil einen ersten bzw. einen zweiten Scheitelpunkt und eine erste bzw. eine zweite Basis aufweisen, wobei der erste und der zweite konische Teil an der ersten und der zweiten Basis verbunden werden, wobei der erste Kragen den ersten Scheitelpunkt des ersten konischen Teils definiert und der zweite Kragen den zweiten Scheitelpunkt des zweiten konischen Teils definiert, wobei das zweite und das dritte Muster Spiegelbilder voneinander sind und wobei das erste Muster vom zweiten und vom dritten Muster unterschiedlich ist und vorausgewählt wird, so dass der Raum zwischen benachbarten Stegen des ersten konischen Teils größer ist als der des zweiten konischen Teils.

## Revendications

1. Dispositif d'occlusion (10; 110; 210) comprenant:
un tube (12 ; 112 ; 212) comportant une extrémité proximale (14 ; 114 ; 214) s'étendant jusqu'à une extrémité distale (16) et une paroi tubulaire (17) définissant une lumière (18 ; 118) formée à travers celui-ci, le tube définissant un premier col (20 ; 120) au niveau de l'extrémité proximale et un second col (22 ; 222) disposé entre les extrémités proximale et distale, une pluralité d'incisions (24 ; 124) formées à travers la paroi tubulaire définissant une pluralité d'éléments structurels (26 ; 126; 226) déployables radialement, la pluralité d'éléments structurels étant sollicités vers un état déployé radialement, la pluralité d'éléments structurels définissant une partie bulbeuse (30; 130) s'étendant entre les premier et second cols et une première partie conique (32 ; 132) s'étendant du second col à l'extrémité distale du tube ;
la première partie conique étant définie par un premier ensemble d'incisions (24 ; 124) formées à travers la paroi tubulaire suivant un premier motif ;
la partie bulbeuse comprenant une deuxième partie conique (30b) et une troisième partie conique (30a), la deuxième partie conique étant définie par un deuxième ensemble d'incisions formées à travers la paroi tubulaire suivant un deuxième motif, et la troisième partie conique étant définie par un troisième ensemble d'incisions formées à travers la paroi tubulaire suivant un troisième motif ;
la deuxième partie conique étant adjacente à la fois à la première partie conique et à la troisième partie conique ;
les deuxième et troisième motifs étant différents de telle sorte que les éléments structurels des deuxième et troisième parties coniques de la partie bulbeuse forment différents motifs, ledit dispositif d'occlusion étant **caractérisé en ce que** le troisième motif est présélectionné de telle sorte que l'espace entre des éléments structurels (26 ; 126 ; 226) adjacents de la troisième partie conique (30a) soit plus large que celui de la deuxième partie conique.

2. Dispositif (10 ; 110 ; 210) selon la revendication 1, dans lequel la partie bulbeuse (30 ; 130) est configurée pour se déployer jusqu'à un premier diamètre maximum (d₁) et la première partie conique (32 ; 132) est configurée pour se déployer jusqu'à un deuxième diamètre maximum (d₂) égal au premier diamètre maximum.

3. Dispositif (10 ; 110 ; 210) selon la revendication 1, dans lequel la première partie conique (32 ; 132) s'étend d'un premier sommet au niveau du second col (22 ; 222) à une première base au niveau de l'extrémité distale (16) du tube (12; 112 ; 212).

4. Dispositif selon la revendication 3, dans lequel la deuxième partie conique (30b) comporte un deuxième sommet au niveau du second col et la troisième partie conique (30a) comporte un troisième sommet au niveau du premier col, les deuxième et troisième parties coniques s'étendant chacune de leurs deuxième et troisième sommets respectifs à des deuxième et troisième bases respectives raccordées l'une à l'autre.

5. Dispositif (10 ; 110 ; 210) selon la revendication 1, dans lequel le deuxième motif de la deuxième partie conique (30b) est une image spéculaire du premier motif de la première partie conique (32 ; 132), et dans lequel le troisième motif est différent à la fois des premier et deuxième motifs.

6. Dispositif (10 ; 110 ; 210) selon la revendication 4, dans lequel le troisième motif de la troisième partie conique (30a) définit des éléments structurels (26 ; 126) s'étendant en arc entre le premier col (20; 120) et la deuxième partie conique (30b), chacun des éléments structurels de la troisième partie conique évitant le contact avec d'autres éléments structurels de la troisième partie conique tandis que les éléments structurels s'étendent du troisième sommet en direction de la troisième base.

7. Dispositif (10 ; 110 ; 210) selon la revendication 1, dans lequel le premier col (20 ; 120) est défini par une première partie sans incision du tube (12 ; 112 ; 212) et le second col (22 ; 222) est défini par une seconde partie sans incision du tube, le premier ensemble d'incisions (24 ; 124) s'étendant du second col à l'extrémité distale (16) du tube, le deuxième ensemble d'incisions s'étendant du second col à la troisième partie conique (30a), et le troisième ensemble d'incisions s'étendant de la deuxième partie conique (30b) au premier col.

8. Dispositif selon la revendication 1, dans lequel le second col (22 ; 222) est défini par une partie sans incision du tube (12 ; 112 ; 212), le premier ensemble d'incisions (24) s'étendant du second col à l'extrémité distale (16) du tube, le deuxième ensemble d'incisions s'étendant du second col à la troisième partie conique (30a), et le troisième ensemble d'incisions s'étendant de la deuxième partie conique (30b) à l'extrémité proximale (14 ; 114 ; 214) du tube, dans lequel les éléments structurels (26) formés par le troisième ensemble d'incisions sont rassemblés au niveau de leurs extrémités proximales afin de définir le premier col (20 ; 120).

9. Dispositif selon la revendication 1, comprenant en outre une membrane d'occlusion (40; 140) fixée aux éléments structurels (26) au niveau des première et deuxième parties coniques (32, 30b).

10. Dispositif selon la revendication 9, dans lequel la membrane d'occlusion (40 ; 140) est fixée à une surface intérieure des éléments structurels au niveau des première et deuxième parties coniques (32, 30b).

11. Dispositif (10 ; 110 ; 210) selon la revendication 4, dans lequel, dans l'état déployé radialement, la première base de la première partie conique (32 ; 132) définit un premier diamètre (d₁), la deuxième base de la deuxième partie conique (30b) définit un deuxième diamètre (d₂), et la troisième base de la troisième partie conique (30a) définit un troisième diamètre (d₂), dans lequel les premier, deuxième et troisième diamètres sont égaux les uns aux autres de telle sorte que la partie bulbeuse (30) et la première partie conique présentent le même diamètre.

12. Dispositif (10 ; 110 ; 210) selon la revendication 1, dans lequel les premier et deuxième motifs des première et deuxième parties coniques (32 ; 132, 30b) respectives sont définis par une pluralité de losanges formés par la pluralité d'éléments structurels (26) de chacune des première et deuxième parties coniques (32 ; 132, 30b).

13. Dispositif (10 ; 110 ; 210) selon la revendication 1, le dispositif pouvant être mis en place sur un fil-guide (80), la lumière (18 ; 118) du tube (12 ; 112 ; 212) étant configurée pour recevoir le fil-guide.

14. Dispositif (210) selon la revendication 1, dans lequel le premier col comprend un mécanisme de fixation (244) permettant de fixer le dispositif à un organe de pose durant la pose du dispositif dans un vaisseau d'un patient.

15. Procédé de fabrication d'un dispositif d'occlusion (10 ; 110 ; 210) comprenant:
pratiquer une pluralité d'incisions (24) à travers une partie d'une paroi tubulaire (17) d'un tube (12 ; 112 ; 212) comportant une extrémité proximale (14 ; 114 ; 214) et une extrémité distale (16), la paroi tubulaire définissant une lumière (18 ; 118) formée à travers les extrémités proximale et distale du tube, le fait de pratiquer une pluralité d'incisions à travers la paroi tubulaire définissant une pluralité d'éléments structurels déployables radialement ; et
déployer la pluralité d'éléments structurels de façon à former une armature comportant un premier col (20 ; 120) au niveau de l'extrémité proximale du tube, une partie bulbeuse (30) s'étendant entre le premier col et un second col (22 ; 222) situé entre les extrémités proximale et distale du tube, et une partie évasée (32 ; 132) s'étendant du second col à l'extrémité distale du tube, le fait de déployer la pluralité d'éléments structurels comprenant le fait de déployer la partie bulbeuse et la partie évasée jusqu'au même diamètre maximum (d₁ ; d₂ ) ;
dans lequel le fait de pratiquer une pluralité d'incisions (24) à travers la paroi tubulaire (17) comprend le fait de pratiquer un premier ensemble d'incisions à travers une première partie de la paroi tubulaire, le fait de pratiquer un deuxième ensemble d'incisions à travers une deuxième partie de la paroi tubulaire, et le fait de pratiquer un troisième ensemble d'incisions à travers une troisième partie de la paroi tubulaire, le premier ensemble d'incisions étant formé suivant un premier motif, le deuxième ensemble d'incisions étant formé suivant un deuxième motif, et le troisième ensemble d'incisions étant formé suivant un troisième motif, la partie évasée (32 ; 132) étant définie par la troisième partie avec le troisième ensemble d'incisions, la partie bulbeuse (30) étant définie par les première et deuxième parties, la première partie avec le premier ensemble d'incisons formant une première partie conique (30a) et la deuxième partie avec le deuxième ensemble d'incisions formant une deuxième partie conique (30b), les première et deuxième parties coniques comportant chacune des premier et deuxième sommets et des première et deuxième bases respectifs, les première et deuxième parties coniques étant raccordées au niveau des première et deuxième bases, le premier col définissant le premier sommet de la première partie conique et le second col définissant le deuxième sommet de la deuxième partie conique, dans lequel les deuxième et troisième motifs sont des images spéculaires l'un de l'autre, et dans lequel le premier motif est différent des deuxième et troisième motifs et est présélectionné de telle sorte que l'espace entre des éléments structurels adjacents de la première partie conique soit plus large que celui de la deuxième partie conique.
